# EUROPÄISCHE PATENTSCHRIFT

(11) **EP 1 853 907 B1**
(45) Veröffentlichungstag und Bekanntmachung des Hinweises auf die Patenterteilung: **20.04.2011**
(21) Anmeldenummer: 06723125.8
(22) Anmeldetag: 27.02.2006
(51) Int. Cl.: G01N 31/12

(54) **VERFAHREN ZUR BESTIMMUNG VON ABBAUBAREM, ORGANISCHEM KOHLENSTOFF**
METHOD FOR THE DETERMINATION OF DEGRADABLE, ORGANIC CARBON
PROCEDE POUR DETERMINER DU CARBONE ORGANIQUE DECOMPOSE

(30) Priorität: 01.03.2005 DE 102005009828
(43) Veröffentlichungstag der Anmeldung: 14.11.2007
(73) Patentinhaber: Elementar Analysensysteme GmbH, 63452 Hanau (DE)
(72) Erfinder: KUPKA, Hans-Joachim, 63543 Neuberg (DE); DUNSBACH, Ralf, 61267 Neu-Anspach (DE)
(74) Vertreter: Grimm, Ekkehard
(86) Internationale Anmeldenummer: PCT/EP2006/001764
(87) Internationale Veröffentlichungsnummer: WO 2006/092253

(56) Entgegenhaltungen:
- DE-C1- 4 436 205
- GB-A- 2 271 179
- US-A- 5 958 777
- MARTIN A E ET AL: "GAS-PHASE CHROMATOGRAPHY" NATURE, NATURE PUBLISHING GROUP, LONDON, GB, Bd. 175, 5. März 1955 (1955-03-05), Seiten 422-423, XP001149201 ISSN: 0028-0836
- MARZI T.; NOWARA N.; BRUISTEN M.: "Direktmessung zur Differenzierung zwischen abbaubarem organischen Kohlenstoff (AOC) und elementarem Kohlenstoff in Anlehnung an die VGB-Methode" MÜLL UND ABFALL, vol. 33, 2001, pages 24-28, XP008110187 ISSN: 0027-2957

## Beschreibung

Die vorliegende Erfindung betrifft ein Verfahren zur Bestimmung von abbaubarem, organischem Kohlenstoff.

Auf Grund der Vorschriften zur Abfallbeseitigung ist für viele Abfallarten die Bestimmung des organischen Kohlenstoffgehaltes entscheidend für die Einstufung in jeweiligen Deponieklassen. Wird der organische Kohlenstoff klassisch nach DIN EN 13137 bestimmt, führt dies zwangsläufig zu einer Erfassung nicht nur des organisch gebundenen Kohlenstoffs, sondern auch des elementar vorliegenden Kohlenstoffs. Dieser elementare Kohlenstoff unterliegt keiner biogenen Veränderung und beeinträchtigt somit nicht die Stabilität des Deponiekörpers. Wünschenswert ist daher ein Analyseverfahren, das eine Unterscheidung zwischen biologisch abbaubarem und elementarem Kohlenstoff erlaubt.

Einen Ansatz für ein solches Analyseverfahren stellt die sogenannte VGB-Methode dar. Nach dieser Methode wird die zu untersuchende Probe zunächst einer Pyrolyse unterzogen, gefolgt von einer Hochtemperaturoxidation des Pyrolyserückstandes. Von dem klassisch bestimmten TOC-Gehalt wird dann der sogenannte Restkohlenstoff (RC) abgezogen und die Differenz ergibt den sogenannten abbaubaren organischen Kohlenstoff (AOC). Da ein Teil des abbaubaren organischen Kohlenstoffs während der Pyrolyse in elementaren Kohlenstoff umgewandelt wird (Pyrolysekoks), wird der AOC-Wert mit einem empirischen Faktor multipliziert. Üblicherweise wird hier ein Wert von 1,3 herangezogen. Diese Methode hat im Laborablauf zwei wesentliche Nachteile:
- der klassische TOC muss separat bestimmt werden; dies bedeutet die Durchführung von zwei Analysedurchläufen und somit einen doppelten Arbeitsaufwand;
- ist RC » AOC, so ist der AOC bedingt durch die Differenzbildung mit einem hohen statistischen Fehler behaftet, da sich die absoluten Fehler der RC- und TOC-Bestimmungen addieren.

Eine an die VGB-Methode angelehnte Direktbestimmung des AOC wird von Marzi et al. beschrieben (Müll und Abfall, Bd. 33 (2001), Seiten 24-28), wobei man die Probe mit Inertgas bei 650°C-850°C behandelt, das entstehende Pyrolysegas verbrennt, und durch Messung des CO₂ Rückschlüsse auf den AOC zieht. Die Bestimmung des RC erfolgt anschließend durch Verbrennung des Pyrolyserückstandes aus der AOC-Messung.

Wünschenswert ist daher, den RC und den AOC in einem Analysenablauf direkt zu bestimmen.

Gelöst wird die Aufgabe durch ein Verfahren gemäß Anspruch 1.

Bei diesem Verfahren wird die Probe bei 850°C zunächst mit Inertgas behandelt. Die Probe kann dabei über einen Greifer, der gleichzeitig als Dosierungslanze für Trägergas ausgebildet und eingesetzt werden kann, dem Verbrennungsrohr, vorzugsweise einem vertikalen Verbrennungsrohr, zugeführt werden. Das entstehende Pyrolysegas, welches den AOC (abbaubaren organischen Kohlenstoff) enthält, wird über ein auf 930°C beheiztes mit CuO als Katalysator gefülltes Rohr geführt. Dort wird der AOC unter Reduktion des CuO zu CO₂ oxidiert und nachfolgend mittels eines IR-Detektor, bevorzugt mittels eines nicht-dispersiven IR-Detektors, gemessen. Wenn der CO₂-Peak abgeklungen ist, wird das Trägergas von Inertgas, vorzugsweise N₂, auf O₂ umgeschaltet. In der O₂-Atmosphäre verbrennt dann der RC (Restkolenstoff), wobei gleichzeitig der Metalloxid-Katalysator, vorzugsweise CuO, regeneriert wird. Mit diesem Verfahren werden in einem Analysengang der AOC und der RC direkt bestimmt, ohne dass eine Probenvorbereitung erforderlich wäre. Diese Verfahrensweise bringt somit erhebliche Vorteile gegenüber dem Stand der Technik mit sich.

Bevorzugt sollte ein vertikal angeordnetes Verbrennungsrohr eingesetzt werden, in das die Probe eingebracht wird. Ein solches vertikal ausgerichtetes Verbrennungsrohr hat für das Verfahren den Vorteil, dass der Probenteil vollständig mit inertgas gespült werden kann.

Um einen automatischen Analysenablauf zu erreichen, sollte die Probe mittels Greifer in das Verbrennungsrohr eingebracht werden. Weiterhin ist hierdurch gewährleistet, dass jede Probe mit der gleichen Geschwindigkeit in das Rohr eingebracht wird, so dass jede Probe den gleichen Temperaturgradienten durchläuft.

Der für die Verbrennung erforderliche Sauerstoff wird über eine Lanze, an deren Ende der Greifer angeordnet ist, unmittelbar auf die Probe zugeführt Hierdurch wird der im Tiegel enthaltene Sauerstoff vor der Analyse entfernt

Als Metalloxid sollte Kupferoxid eingesetzt werden; Kupferoxid ist ein hoch effektiver Sauerstoffdonor über einen weiten Temperaturbereich.

Falls angesäuerte Proben analysiert werden sollen, wird als Metalloxid vorzugsweise Wolf ramoxid eingesetzt.

Cerdioxid sollte als das Metalloxid dann eingesetzt werden, wenn in der Probenmatrix hohe Chloridgehalte vorliegen.

Das CO₂ wird mittels IR-Detektor gemessen, da ein IR-Detektor selektiver ist als ein coulometrischer Detektor. Der Nachweis organischen Materials durch Verbrennung und anschließende CO₂-Messung im IR-Detektor ist im Prinzip bekannt, z.B. DE4436205 und Martin et al. (Nature 175 (1955), 422f).

Nachfolgend wird das erfindungsgemäße Verfahren unter Heranziehung der Zeichnungen beschrieben. In den Zeichnungen zeigt
Figur 1 einen schematisch dargestellten apparativen Aufbau zur Durchführung des Analyseschritts,
Figur 2 den schematisch in Figur 1 dargestellten apparativen Aufbau zur Durchführung des Verbrennungsschritts, und
Figur 3 ein Zeitdiagramm, in dem die Mess-Peaks dargestellt sind.

Als Gerätebasis für die Durchführung des erfindungsgemäßen Verfahrens kann der Elementar-Analysator "varioMAX CN" der Anmelderin, der elementar Analysensysteme GmbH, 63452 Hanau, eingesetzt werden, so dass in Bezug auf Gerätedetails, die in der nachfolgenden Beschreibung nicht im Einzelnen angeführt sind, auf diesen Analysator verwiesen wird.

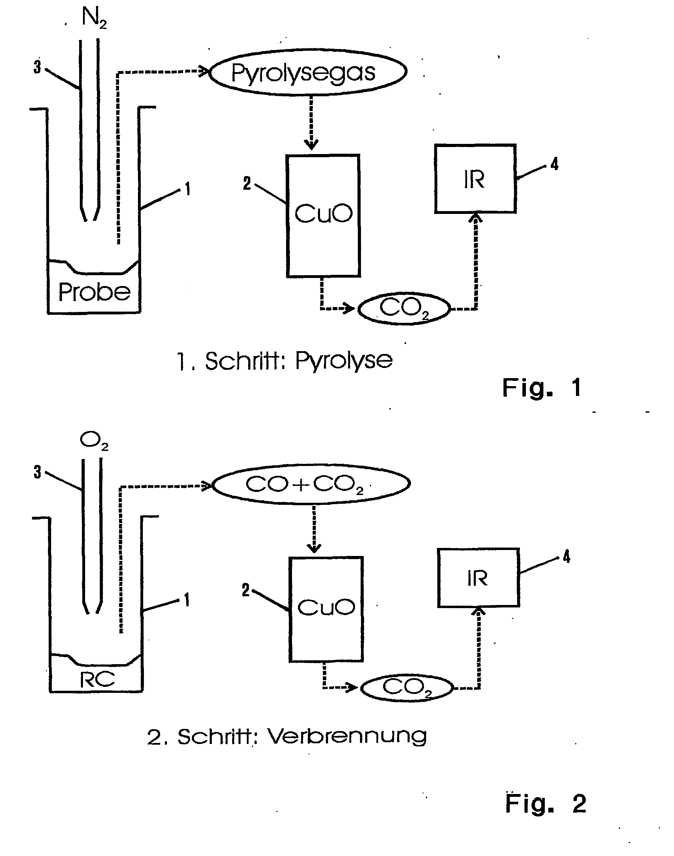

Kernstück der Vorrichtung ist eine Verbrennungs/Pyrolyseanordnung, wie sie schematisch in Figur 1, ohne Verbrennungsrohre, dargestellt ist.

Die nicht dargestellte Verbrennungseinheit umfasst zwei Verbrennungsrohre, die nacheinander angeordnet sind. Das erste Verbrennungsrohr ist im unteren Bereich mit CuO als Katalysator gefüllt ist, während der obere Bereich der Aufnahme des Probentiegels dient, der in der Figur 1 gezeigt und mit dem Bezugszeichen 1 bezeichnet ist.. Das zweite Verbrennungsrohr ist mit CuO als Oxidationsmittel gefüllt, das in der Figur 1 mit dem Bezugszeichen 2 bezeichnet ist. Das erste Rohr (Pyrolyse- und Verbrennungsrohr) wird bei 850°C betrieben, während das zweite Rohr (Nachoxidation) bei 930°C betrieben wird.

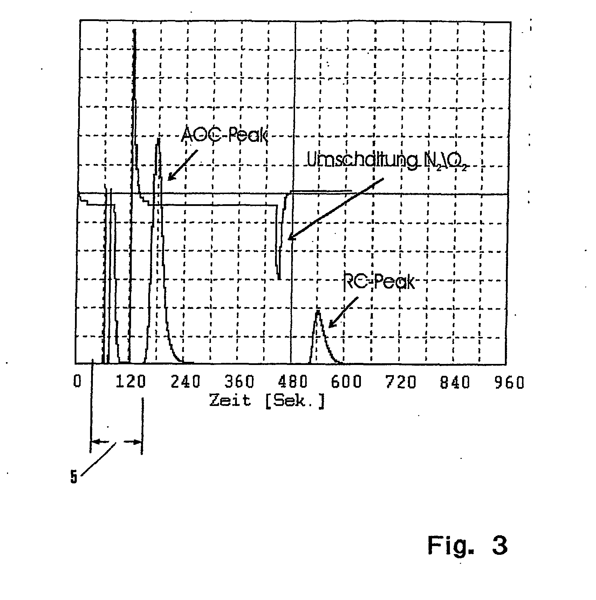

Die Probe, die in den Tiegel 1 eingefüllt ist, wird automatisch mittels eines nicht näher dargestellten Greiferarms in das erste Verbrennungs/Pyrolyserohr eingeführt. Die Gaszuführung, durch die Lanze 3 angedeutet, erfolgt dabei über den Greifer, in den die dargestellte Lanze 3 integriert ist, so dass die Probe bereits vor der Einführung in den Ofen mit dem jeweiligen Trägergas (N₂) beaufschlagt wird. Die Ofentemperatur ist einstellbar, wobei durch die Verwendung von CuO als Katalysatormaterial die obere Grenze auf ca. 900°C festlegt wird. Liegt dann Inertgas an, so wird der AOC in die Gasphase überführt und an dem CuO zu CO₂ oxidiert; hierbei wird das CuO zu Cu reduziert. Das CO₂ wird dann in einem IR-Detektor 4 mit einem entsprechenden Peak gemessen, der in dem Diagramm der Figur 3 dargestellt ist

Ist dieser erste CO₂-Peak abgeklungen, wird das Inertgas durch Sauerstoff ersetzt, wiederum über die Lanze 3 direkt auf den Restkohlenstoff (RC) zugeführt, wie dies in Figur 2 angedeutet ist. Hierdurch wird der im Tiegel 1 verbliebene Restkohlenstoff ebenfalls zu CO₂ umgesetzt und analog gemessen. Durch den Überschuss an O₂ wird darüber hinaus die CuO-Füllung im Verbrennungsrohr regeneriert.

Auf diese Weise erhält man, wie dem Diagramm der Figur 3 zu entnehmen ist, zwei deutlich voneinander getrennte Peaks für abbaubaren organischem Kohlenstoff (AOC-Peak) und inertem Kohlenstoff (RC-Peak). Wie der Zeitskala (x-Achse) der Figur 3 zu entnehmen ist, benötigt der gesamte Messvorgang einschließlich der Probenzuführung (der mit dem Bezugszeichen 5 gekennzeichnete Bereich etwa 10 Minuten.

Unverbrennbare Bestandteile verbleiben in dem Tiegel 1, der nach der Analyse in einen Vorratsbehälter fällt. Der Tiegel 1 selbst kann nach Entleerung wiederverwendet werden.

Dieser Messablauf kann eingesetzt werden, wenn der Gehalt, an TIC (Karbonate und Hydrogenkarbonate) gegenüber dem TOC vernachlässigbar ist. Sollte das nicht der Fall sein, so kann die Probe im Vorwege mittels HCl angesäuert werden. Die Probe ist danach im Trockenschrank für 2h bei 80°C zu trocknen, um die überschüssige HCl auszutreiben. Danach kann die Probe dann wie oben beschrieben gemessen werden. Alternativ dazu kann der TIC separat bestimmt und von dem AOC-Wert abgezogen werden. Versuche mit Ca-CO₃ haben gezeigt, dass der TIC vollständig während der Pyrolysephase zu CO₂ umgesetzt wird.

### Messergebnisse

Mit dem beschriebenen Aufbau und der vorstehend angegebenen Verfahrensweise wurden unterschiedliche Proben und Reinsubstanzen vermessen. Bei der Bestimmung der Reinsubstanzen muß ein Korrekturfaktor herangezogen werden der, aufgrund umfangreicher Untersuchungen mit 1,3, für die meisten organischen Verbindungen ermittelt wurde, der für den AOC anzuwenden ist. Mit diesem Korrekturfaktor von 1,3 ist der Wert für den AOC zu multiplizieren. Eine Ausnahme von der vorstehenden Vorschrift stellt die eine von der Fa. Fluka bezogene Huminsäure dar, bei der sich der Korrekturfaktor unter der Annahme, dass diese Huminsäure keinen elementaren Kohlenstoff enthält, zu 2,6 errechnet. In der nachfolgenden Tabelle sind die Messergebnisse verschiedener Substanzen, die umfangreich geprüft wurden, aufgelistet.

| Substanz | AOC [Masse-%] | RC [Masse-%] | AOC theor. [Masse-%] | Faktor |
|---|---|---|---|---|
| Asparaginsäure | 26,0 | 10,1 | 36,1 | 1,39 |
| Glutaminsäure | 30,9 | 9,9 | 40,78 | 1,32 |
| Huminsäure | 17,3 | 28,0 | 45,3 | 2,62 |
| Saccharose | 31,6 | 10,5 | 42,1 | 1,33 |

Es wurden weiterhin diverse Abfallarten und industrielle Nebenprodukte auf elementaren Kohlenstoff mit dem erfindungsgemäßen Verfahren untersucht. Erwartungsgemäß überwiegt bei Klärschlamm der organische Anteil, während der Kohlenstoff aus Verbrennungsschlacken überwiegend elementar vorliegt. Auch Böden weisen einen erheblichen Anteil an elementarem Kohlenstoff auf, was insofern logisch erscheint, als dass anthropogen beeinflusste Oberböden (und in der Abfalldiskussion geht es hauptsächlich um diese) häufig mit Verbrennungsschlacken versetzt sind.

Dabei zeigte das erfindungsgemäße Verfahren durchweg gute Reproduzierbarkeiten, nicht nur in bezug auf den Parameter TOC (d.h., die Summe aus AOC und RC), sondern auch in Bezug auf beide Einzelparameter. Der Pyrolysevorgang läuft also kontrolliert und reproduzierbar ab. Dies wird durch die nachfolgend aufgeführten Messergebnisse bestätigt.

| Probe | AOC [Masse-%] | SDr [%] | RC [Masse-%] | SDr [%] | AOC korr. [Masse-%] |
|---|---|---|---|---|---|
| Klärschlamm | 22,8 | 2,4 | 9,71 | 1,85 | 29,7 |
| Verbrennungsschlakke | 1,29 | 3,27 | 9,73 | 0,63 | 1,64 |
| Kohle | 15,7 | 3,42 | 49,1 | 1,32 | 20,6 |
| Boden | 13,2 | 1,80 | 25,1 | 1,08 | 17,2 |

Somit stellt das beschriebene Verfahren mit der entsprechenden Vorrichtung eine erhebliche Vereinfachung für die Durchführung der VGB-Methode dar. Die automatische Probenzuführung sowie die Bestimmung von AOC und RC reduzieren die Analysenzeit auf von ca.60 Minuten auf 10 Minuten.

## Patentansprüche

1. Verfahren zur Bestimmung von abbaubarem, organischem Kohlenstoff (AOC) mit folgenden Verfahrensschritten:
- Zuführen der Probe zu einem ersten Verbrennungsrohr (1) unter Zuführung eines Trägergases über eine Lanze (3),
- Behandeln der Probe bei mindestens 850°C mit Inertgas im ersten Verbrennungsrohr (1).
- Leiten des entstehenden Pyrolysegases, welches den abbaubaren, organischen Kohlenstoff (AOC) enthält, über ein Metalloxid (2) in einem zweiten Verbrennungsrohr als Sauerstoffdonor ohne Zugabe von Sauerstoff zu Oxidationszwecken,
- Oxidieren des abbaubaren, organischen Kohlenstoffs (AOC) in dem zweiten Verbrennungsrohr unter Reduktion des Metalloxids und
- Messen des abbaubaren, organischen Kohlenstoffs (AOC) mittels eines IR-Detektors (4) unter Erfassen des CO₂-Peaks,
nach Abklingen des CO₂-Peaks, Umstellen des Trägergases von Inertgas auf O₂, wobei O₂ über dieselbe Lanze (3) zugeführt wird, über die auch das Inertgas zugeführt wird,
- Verbrennen des Restkohlenstoffs (RC) in dem ersten Verbrennungsrohr (1) in der O₂-Atmosphäre unter gleichzeitiger Regenerierung des Metalloxids in dem zweiten Verbrennungsrohr und
- Messen des Restkohlenstoffs (RC) mittels IR-Detektor (4).

2. Verfahren nach Anspruch 1, **dadurch gekennzeichnet, dass** die Probe in ein vertikal angeordnetes Verbrennungsrohr (1) eingebracht wird.

3. Verfahren nach Anspruch 2, **dadurch gekennzeichnet, dass** die Probe mittels Greifer in das Verbrennungsrohr (1) eingebracht wird.

4. Verfahren nach Anspruch 3, **dadurch gekennzeichnet, dass** Sauerstoff über eine Lanze (3), an deren Ende ein Greifer angeordnet ist, unmittelbar auf die Probe zugeführt wird.

5. Verfahren nach einem der Ansprüche 1 bis 4, **dadurch gekennzeichnet, dass** als Metalloxid Kupferoxid (2) eingesetzt wird.

6. Verfahren nach einem der Ansprüche 1 bis 4, **dadurch gekennzeichnet, dass** als Metalloxid Wolframoxid eingesetzt wird.

7. Verfahren nach einem der Ansprüche 1 bis 4, **dadurch gekennzeichnet, dass** als Metalloxid Cerdioxid eingesetzt wird.

## Claims

1. A method for determining degradable organic carbon (AOC), comprising the following steps:
- feeding the sample to a first combustion tube (1) together with a carrier gas via a lance (3);
- treating the sample with inert gas in the first combustion tube (1) at at least 850°C;
- conducting the obtained pyrolysis gas, which contains the degradable organic carbon (AOC), across a metal oxide (2) in a second combustion tube as an oxygen donor without addition of oxygen for oxidizing purposes;
- oxidizing the degradable organic carbon (AOC) in the second combustion tube while the metal oxide is reduced; and
- measuring the degradable organic carbon (AOC) by means of an IR detector (4) while the CO₂ peak is detected;
switching the carrier gas from inert gas to O₂ once the CO₂ peak has decayed, with O₂ being supplied via the same lance (3) via which the inert gas is also supplied;
- burning the residual carbon (RC) in the first combustion tube (1) in the O₂ atmosphere while the metal oxide is regenerated in the second combustion tube; and
- measuring the residual carbon (RC) by means of an IR detector (4).

2. The method according to claim 1, **characterized in that** the sample is introduced into a vertically arranged combustion tube (1).

3. The method according to claim 2, **characterized in that** the sample is introduced into the combustion tube (1) by means of a gripper.

4. The method according to claim 3, **characterized in that** the oxygen is directly fed to the sample via a lance (3) at the end of which a gripper is arranged.

5. The method according to any one of claims 1 to 4, **characterized in that** the metal oxide is copper oxide (2).

6. The method according to any one of claims 1 to 4, **characterized in that** the metal oxide is tungsten oxide.

7. The method according to any one of claims 1 to 4, **characterized in that** the metal oxide is cerium dioxide.

## Revendications

1. Procédé pour la détermination de carbone organique assimilable (COA) présentant les étapes suivantes de procédé :
- amenée de l'échantillon à un premier tube de combustion (1) avec apport de gaz porteur par une lance (3),
- traitement de l'échantillon à au moins 850°C avec du gaz internet dans le premier tube de combustion (1),
- guidage du gaz de pyrolyse produit contenant le carbone organique assimilable (COA) par un oxyde métallique (2) dans un second tube de combustion en tant que donneur d'oxygène sans ajout d'oxygène dans un but d'oxydation,
- oxydation du carbone organique assimilable (COA) dans un second tube de combustion sous réduction de l'oxyde métallique et
- mesure du carbone organique assimilable (COA) au moyen d'un détecteur IR (4) en détectant les pics CO₂, après retombée du pic CO₂, passage du gaz porteur de gaz interne à l'O₂, l'O₂ étant amené par la même lance (3) qui amène également le gaz inerte,
- combustion du carbone résiduel (CR) dans le premier tube de combustion (1) dans une atmosphère O₂ sous régénération simultanée de l'oxyde métallique dans le second tube de combustion et
- mesure du carbone résiduel (CR) au moyen du détecteur IR (4).

2. Procédé selon la revendication 1, **caractérisé en ce que** l'échantillon est amené dans un tube de combustion (1) vertical.

3. Procédé selon la revendication 2, **caractérisé en ce que** l'échantillon est amené dans le tube de combustion (1) au moyen de pinces.

4. Procédé selon la revendication 3, **caractérisé en ce que** l'oxygène est amené directement sur l'échantillon par une lance (3) à l'extrémité de laquelle est disposée une pince.

5. Procédé selon l'une des revendications 1 à 4, **caractérisé en ce qu'**il est utilisé de l'oxyde de cuivre (2) en tant qu'oxyde de métal.

6. Procédé selon l'une des revendications 1 à 4, **caractérisé en ce qu'**il est utilisé de l'oxyde de tungstène en tant qu'oxyde de métal.

7. Procédé selon l'une des revendications 1 à 4, **caractérisé en ce qu'**il est utilisé de l'oxyde de cérium en tant qu'oxyde de métal.
